## Europäisches Patentamt

## European Patent Office

## Office européen des brevets

(19)

(11) Veröffentlichungsnummer: **0 278 013**
**B1**

(12) **EUROPÄISCHE PATENTSCHRIFT**

(45) Veröffentlichungstag der Patentschrift:
**27.12.89**

(21) Anmeldenummer: **87101630.9**

(22) Anmeldetag: **06.02.87**

(51) Int. Cl.⁴: **A61K 31/505**
**// (A61K31/505, 31:40)**

(54) **Antihypoxisches Mittel.**

(43) Veröffentlichungstag der Anmeldung:
**17.08.88 Patentblatt 88/33**

(45) Bekanntmachung des Hinweises auf die Patenterteilung:
**27.12.89 Patentblatt 89/52**

(84) Benannte Vertragsstaaten:
**BE CH DE ES FR GB IT LI NL SE**

(56) Entgegenhaltungen:
**UNLISTED DRUGS, Band 33, Nr. 12, Dezember 1981, Seite 191m, Chatham, New Jersey, US; "CENTRACETAM"**
**CHEMICAL ABSTRACTS, Band 100, Nr. 9, 27. Februar 1984, Seite 35, Zusammenfassung Nr. 61583x, Columbus, Ohio, US; D. MILANOVA et al.: "Study on the antihypoxic effect of some drugs used in the pharmacotherapy of cerebrovascular disease", & METHODS FIND. EXP. CLIN. PHARMACOL. 1983, 5(9), 607-12 000**

(73) Patentinhaber: **S O "PHARMACHIM", Iliensko Chaussée 16, Sofia(BG)**

(72) Erfinder: **Genova, Ani Ivanova, 17, Sveta Gora St., Sofia(BG)**
Erfinder: **Nikolova, Milka Petrova, 3, Raiko Daskalov St., Sofia(BG)**
Erfinder: **Todorova, Maria Georgieva, 18-A Blvd.Lenin, Sofia(BG)**
Erfinder: **Monov, Alexander Petrov, 24, Midjur St., Sofia(BG)**
Erfinder: **Alexandrov, Nikola Georgiev, Komplex Mladost-3, Sofia(BG)**
Erfinder: **Nikolov, Rumen Kostov, 12, Rozova Dolina St., Sofia(BG)**
Erfinder: **Andonova, Violeta Hinova, Bl.68-A.Komplex Tolstoi, Sofia(BG)**
Erfinder: **Zoneva, Nadejda Ivanova, 11, Blvd.Graf Ignatlev, Sofia(BG)**
Erfinder: **Nisimov, Yosif Nisim, 3, Sofronii St., Sofia(BG)**
Erfinder: **Vitkova, Snejana Georgleva, Bl.1252-2, N.Kamenov St., Sofia(BG)**
Erfinder: **Boyadjiev, Nikola Georglev, 1, Blvd.Samokov, Sofia(BG)**
Erfinder: **Stoyanova, Anastasia Mihailova, Bl 17-2, Komplex Nadejda-6, Sofia(BG)**
Erfinder: **Savova, Keranka Nikolova, Bl.43-2, Komplex Mladost-1, Sofia(BG)**
Erfinder: **Firkova, Nevena Lyubenova, Bl.65 Postoyanstovo St., Sofia(BG)**
Erfinder: **Spasova, Slava Nikolova, 56, Sinmeon Slavkow St., Sofia(BG)**

(74) Vertreter: **von Füner, Alexander, Dr. et al, Patentanwälte v. Füner, Ebbinghaus, Finck Mariahilfplatz 2 & 3, D-8000 München 90(DE)**

## Beschreibung

Die Erfindung betrifft ein antihypoxisches Mittel, insbesondere ein Mittel mit einer gehirnprotektiven und hepatoprotektiven Wirkung, das bei der Behandlung von akuten und chronischen Erkrankungen und Vergiftungen, durch welche der ganze Organismus komplex angegriffen wird, einsetzbar ist.

Es ist bekannt, daß die Orotsäure, die eine Uracil-4-Carbonsäure, bzw. Vitamin $B_{13}$, darstellt, eine Schutzwirkung bei den unterschiedlichen Leberintoxikationen und -beschädigungen aufweist, da sie eine vitaminähnliche Wirkung ausübt und die anabolischen, detoxischen und regenerativen Funktionen der Leberzelle unterstützt. Die Orotsäure weist eine schwache antihypoxische Wirkung auf (Krug, M et al, Psychopharmacol., 1977, 53, 73-78.).Bekannt ist auch, daß das 2-Oxopyrrolidin-1-Acetamid (Piracetam) ein neotropes Präparat darstellt, das eine günstige Wirkung auf krankheitsbedingte Störungen der Gedächtniskraft bei den unterschiedlichsten Beschädigungen des zentralen Nervensystems, wie Gedächtnisstörungen, Gedächtnisschwund, Geistesmüdigkeit, komatöse Zustände und akute psychische Störungen intoxischer Herkunft, alkoholisches Delirium und andere ausübt. (GB. Patentschrift Nr. 1 039 113).

Bei ihrer Anwendung weist die Orotsäure eine schwache antihypoxische Wirkung auf das Leber-Parenchym und eine noch schwächere Wirkung auf das zentrale Nervensystem auf.

Aufgabe der Erfindung war es nun, ein antihypoxisches Mittel zu erarbeiten, insbesondere ein eine gehirnprotektive und hepatoprotektive Wirkung aufweisendes Mittel und ein Verfahren zu seiner Anwendung bei akuten und chronischen Erkrankungen kombinierter Art, wobei das Mittel eine erhöhte antihypoxische Wirkung besitzen soll.

Die Aufgabe wurde durch ein antihypoxisches Mittel, insbesondere durch ein Mittel mit einer gehirnprotektiven und einer hepatoprotektiven Wirkung gelöst, welches Piracetam und Orotsäure und/oder deren pharmazeutisch verträglichen Salze in Gewichtsverhältnissen von 500 : 1 bis 1 : 2 enthält.

Es wurde festgestellt, daß bei einer gleichzeitigen Anwendung von Piracetam und Orotsäure eine äußerst günstige Wechselwirkung zustandekommt, wobei eine größere antihypoxische Wirkung beobachtet wird als bei der getrennten Anwendung der einzelnen Präparate. Die synergistische Wirkung der kombination besteht erfindungsgemäß im Potenzieren der antihypoxischen Wirkung, was durch die Ergebnisse der pharmakologischen Untersuchungen (siehe Tabelle 1) sowie auch durch klinische Versuche festgestellt wurde.

Das erfindungsgemäße Mittel soll vorzugsweise als fertige Arzneiform in den Organismus eingeführt werden.

Die erfindungsgemäße Kombination aktiver Stoffe ist für die Zubereitung von pharmazeutischen Gemischen und Präparaten geeignet. Die fertigen Arzneiformen können die Wirkstoffe und/oder deren pharmazeutisch anwendbare Salze enthalten, gegebenenfalls in einem Gemisch mit den in der Pharmazie üblichen Hilfmitteln und Trägern.

Die erfindungsgemäßen Präparate können oral oder parenteral angewandt werden, wobei die parenterale Aufnahme bei der Behandlung schwerer Erkrankungen und akuter exogener Vergiftungen zu bevorzugen ist. Die orale Therapie in Form von Tabletten, Kapseln, Dragees, Lösungen und Emulsionen ist für leichtere Fälle exogener Vergiftungen, zu prophylaktischen Zwecken oder nach parenteraler Aufnahme im akuten Erkrankungsstadium geeignet.

Einmal tägliche Dosen von Piracetam können von 0,05 bis 5 g und von Orotsäure von 0,01 bis 0,1 g betragen, und 24-stündig - entsprechend von 0,150 bis 50 g und von 0,030 bis 0,30 g - verteilt auf drei bis sechs Verabreichungen.

Prüfungen auf akute Toxizität des erfindungsgemäßen Präparats zeigen, daß seine Anwendung in Dosen von 2500 mg/kg intramuskulär, von 1000mg/ kg intravenös und 3000mg/kg intraperitoneal zu keinen toxischen Effekten führt, was dafür spricht, daß es praktisch nicht toxisch ist. Die pathomorphologischen Prüfungen während einer einmonatigen subchronischen Toxizität an mit 100 mg/kg und 500 mg/kg intramuskulär behandelten Ratten zeigen keine pathologischen Änderungen der Innenorgane und der hämatologischen Kennwerte. Das Präparat weist keinen lokal reizenden Effekt an der Aufnahmestelle auf.

Das erfindungsgemäß Präparat wurde bei der Behandlung an 15 Kranken mit akuten Vergiftungen mit pflanzlichen Toxinen von phalloiden Pilzen erprobt, welche klinisch und labormäßig mit einer toxischen Schädigung der Leber verlaufen sind. Die Ergebnisse sind in den Beispielen angegeben.

Es wurde festgestellt, daß das erfindungsgemäße Mittel bei einer ganzen Reihe heutiger Erkrankungen und Vergiftungen, sowie auch bei manchen komplexen Einwirkungen auf den Organismus des Menschen ökologischer Art Anwendung finden kann, wenn pathologische Veränderungen und Abweichungen vorhanden sind, die sich durch eine akute und chronische Hypoxie auszeichnen, das zentrale Nervernsystem angreifen, wodurch eine gleichzeitige Behandlung der Leber und des Gehirns erforderlich ist.

Die Vorteile des erfindungsgemäßen Mittels bestehen im folgenden: Es wird eine größere antihypoxische Wirkung erzielt, welche sich sowohl bei Gehirn- als auch bei Leberverletzungen zeigt, die bei Erkrankungen und akuten und chronischen Vergiftungen auftreten.

Die Anwendung des Präparats als fertige Arzneiform bietet die Möglichkeit, für eine schnelle und rechtzeitige Verabreichung unterschiedlicher Dosen der Komponenten im günstigsten synergistischen Verhältnis, wobei die Möglichkeit für die Entwicklung einer Nebenwirkung, der sich bei vielen Lebererkrankungen und Vergiftungen bildenden endogenen Produkte vermindert wird, welche sekundär die Gehirnstrukturen beschädigen. Durch die Anwendung des erfindungsgemäßen Mittels wird die durchschnittliche 24-stündige Piracetam-Dosis mit 30 % im Vergleich zu seiner getrennten Anwendung vermindert. Die allgemeine ärztliche Betreuung wird verkürzt und die Gefahr von Komplikationen verhindert.

<u>Beispiel 1</u>

Pharmakologischer Test. Antihypoxischer Effekt nach einem Modell der hypobaren Hypoxie bei Mäusen / Nakani-shi-Verfahren/. Die Versuche wurden an 60 weißen Mäusen Linie H (in Gruppen von je 10 Tieren eingeteilt) in einem hermetischen Glasgefäß durchgeführt, wonach der Luftdruck mittels einer Vakuumpumpe bis zu 210 mm Hg kraß vermindert wird, was einer Höhe von 10 000 m über dem Meeresspiegel entspricht. Das Präparat wird intraperitoneal 30 Minuten vor dem Eintreten der Hypoxie verabreicht. Die Lebensdauer der Mäuse wird in Sekunden gemessen. Die Ergebnisse der Versuchsgruppen werden als prozentuelle Veränderung gegenüber der Kontrollgruppe (Tabelle 1) angegeben.

Aus der Tabelle ist ersichtlich, daß bei einer Kombination von 500 mg/kg Piracetam und 25 mg/kg Orotsäure der höchste additive antihypoxische Effekt von 42,6 % erreicht wird, während bei einer Kombination von 1000 mg/kg Piracetam und 50 mg/kg Orotsäure der höchste additive antihypoxische Effekt von 43,9 % erreichbar ist.

<u>Beispiel 2</u> Klinische Daten

G.D.G. 19 Jahre alt, Nr. 21648/1984, Medizinisches Institut für ärztliche Soforthilfe "N. Pirogow", aufgenommen am 04.10.1984 und entlassen am 05.11.1984. Diagnose: Mycetismus Intox. Phaloides, Gastroentero-colitis acuta, Hepatopatia toxica, Encephalopatia toxica. Anamnese, Status und Untersuchungen: Der Patient hatte mit drei Freunden wilde Pilze gegessen, welche auf offenem Feuer gebraten wurden. Nach einer latenten Periode von 6-7 Stunden begann die Vergiftung mit einer Gastroenterokolitis - Ekel, Erbrechen, Durchfall (Diar-rhöe), allgemeinen toxischen Erscheinungen - Schwäche, Schwindel, Kraftlosigkeit und hepatotoxischen Erscheinungen - Schwere und Schmerzen unter der rechten Rippe, Vergrößerung der Leber unter dem Rippenbogen mit 3 - 4 cm. Der Puls des Kranken betrug bei der Aufnahme im Krankenhaus 108 pro Minute bei einem Blutdruck von 120/80.

Nachstehend einige Daten der Untersuchungen:

|  | 04.10.84 | 05.10.84 | 06.10.84 | 10.10.84 | 31.10.84 |
|---|---|---|---|---|---|
| SGOT | 80 me/l | 86 | 22 | 11 | 6 |
| SGPT | 23 me/l | >86 | 77 | 58 | 2 |
| Serum-Bilirubin | 39,31 mkmol/l | 27,9 | 18,6 | 25,9 | 17 |

Die Behandlung bestand in einer Magenspülung , forcierter Diurese, hyperbarer Oxyenase, allgemeinen antitoxischen Mitteln und Piracetam in einer Dosis von bis zu 4 g täglich und 1%iger Orotsäure-Lösung je 1 ml intramuskulär. Der Patient wurde als klinisch gesund entlassen

<u>Beispiel 3</u>

I.T.N. 19 Jahre alt, Nr. 21650/1984, Medizinisches Institut für ärztliche Soforthilfe "N. Pirogow", aufgenommen am 04.10.1984 und entlassen am 19.10.1984.
Diagnose: Mycetismus Intox. phaloides, Gastroentero-colitis acuta, Hepatopatia toxica, Encephalopatia toxica.

Aus der Anamnese, Status und Untersuchungen: Der Patient hatte mit drei Freunden wilde Pilze gegessen, welche auf offenem Feuer gebraten wurden. Nach einer latenten Periode von 7 - 8 Stunden begann sich die Vergiftung mit einer Enterokolitis - Ekel, Erbrechen, Durchfall, allgemeinen toxischen Erscheinungen - Schwäche, Schwindel und hepatotoxischen Erscheinungen - Schwere und Schmerzen unter der rechten Rippe, Vergrößerung der Leber unter dem Rippenbogen mit 2 - 3 cm bemerkbar zu machen. Bei der Aufnahme ins Krankenhaus betrug der Puls des Kranken 96 pro Minute bei einem Blutdruck von 100/80.

Nachstehend einige Daten der Untersuchungen:

|              | 04.10.84       | 05.10.84 | 06.10.84 | 07.10.84 | 18.10.84 |
|--------------|----------------|----------|----------|----------|----------|
| SGOT         | 35 me/l        | 47       | 10       | 17       | 4        |
| SGPT         | 33 me/l        | 37       | 40       | 7        | 2        |
| Serumbilirubin | 32,07 mkmol/l | 10,4    | 7,2      | 11,4     | 13,4     |
| Urämie       | 8,09 mkmol/l   | –        | –        | –        | 4,3      |

Der Patient wurde mit einer Magenspülung, forcierter Diurese, hyperbarer Oxygenase, allgemeinen antitoxischen Mitteln, Piracetam in einer Dosis bis zu 4 g täglich und Orotsäure, und zwar deren 1%ige Lösung je 1 ml injiziert intramuskulär 2 mal während 10 Tagen, behandelt. Der Patient wurde als klinisch gesund entlassen.

Beispiel 4

S.S.T. 19 Jahre alt, 21651/1984, Medizinisches Institut für ärztliche Soforthilfe "N. Pirogow", aufgenommen am 04.10.1987 und entlassen am 24.10 1984.
Diagnose: Mycetismus Intox. phaloides, Gastroenterokolitis acuta, Hepatopatia toxica, Encephalopatia toxica. Wegen der Gleichartigkeit der Daten der Anamnese und des Status mit den obenangegebenen werden nur die Daten einiger charakteristischer Prüfungen aufgeführt.

|              | 04.10.84       | 06.10.84 | 07.10.84 | 11.10.84 | 22.10.84 |
|--------------|----------------|----------|----------|----------|----------|
| SGOT         | 24 me/l        | 43       | 36       | 13       | 10       |
| SGPT         | 33 me/l        | 86       | 72       | 79       | 4        |
| Serumbilirubin | 18,1 mkmol/l | 30,19    | 10,3     | 14,3     | 6,2      |

Die Behandlung des Patienten bestand in Magenspülung, forcierter Diurese, hyperbarer Oxygenase, allgemeinen antitoxischen Mitteln und Piracetam 4 g pro Tag und Orotsäure (1%ige Lösung) 2 Mal je 1 ml intramuskulär während 10 Tagen. Der Kranke wurde als klinisch gesund entlassen.

Beispiel 5

I.E.I. 19 Jahre alt, 21647/1984. Medizinisches Institut für ärztliche Soforthilfe "N. Pirogow", aufgenommen am 04.10.84 und entlassen am 19.10.84.
Diagnose: Mycetismus Intox. phaloides, Gastroenterokolitis actua, Hepatopatia Toxica.
Wegen der Gleichartigkeit der Daten der Anamnese und des Status mit den obenangegebenen Beispielen 2 und 3 werden hier nur die Daten einiger charakteristischer Prüfungen aufgeführt.

|              | 04.10.84       | 05.10.84 | 06.10.84 | 07.10.84 | 11.10.84 |
|--------------|----------------|----------|----------|----------|----------|
| SGOT         | 80 me/l        | über 80  | 16       | 24       | 10       |
| SGPT         | 46 me/l        | über 86  | 36       | 23       | 33       |
| Serumbilirubin | 28,08 mkmol/l | 16,55   | 32,1     | 25,9     | 14,5     |
| und am 18.10.84 entsprechend 4,5 und 15,5 |

Die Behandlung des Patienten bestand in Magenspülung, forcierter Diurese, hyperbarer Oxygenase, allgemeinen antitoxischen Mitteln und 4 g Piracetam pro Tag und Orotsäure (1%ige Lösung) zweimal je 1 ml intramuskulär während 10 Tagen. Der Kranke wurde als klinisch gesund entlassen

Beispiel 6

Ergebnisse der klinischen Prüfung des Orozetams in der Zeit vom 05.08.1985 bis 04.12.1985 in der II. Nervenklinik, NINPN und der Medizinischen Akademie. Es wurden 32 Kranke behandelt, davon 21 in Verhältnissen eines akuten und eines chronischen Versuches und 11 in einem chronischen Versuch.
Die Verteilung der behandelten Kranken ist dem Geschlecht und Alter nach in Tabelle 1 dargestellt.

Tabelle 1

| Geschlecht | Altersgruppen | | | |
|---|---|---|---|---|
| | bis 40 | 41–50 | 51–60 | >60 |
| Männer | 2 | 5 | 5 | 2 |
| Frauen | 4 | 9 | 4 | 1 |
| insgesamt | 6 | 14 | 9 | 3 |

Die Verteilung nach nosologischen Einheiten ist in Tabelle 2 veranschaulicht.

Tabelle 2

| | |
|---|---|
| Im Zustand nach einem Gehirnschlag | 5 |
| Chronische und zeitweilige Gehirn-Gefäßinsuffizienz | 14 |
| Vasomotorische Zephalalgie | 10 |
| Im Zustand nach einem Schädel-Gehirntrauma | 2 |
| im Zustand nach einer Virus-Meningitis | 1 |
| insgesamt | 32 |

Methodik

a/ bei dem akuten Versuch, durchgeführt an 21 Kranken: Nach einer Aufzeichnung auf Phonorheogrammen - (ein volumetrisches und differentiales (Geschwindigkeits-) rechtsseitiges frontomastoides und ein volumetrisches und Geschwindigkeits- longitudinales) wurde im linken Unter-schenkel intramuskulär 5 ml Orozetam injiziiert. Die rheographischen Veränderungen wurden bei denselben Ableitungen in der 5., 15., 30., 60. und 120. Minute nach dem Injizieren des Präparats verfolgt.

Bei der Quantitätsanalyse wurden folgende Kennwerte benutzt: Amplitude der rheographischen Welle /$H_1$/, dikrotischer und diastolischer Index, relativer Teil der Anakrotie, Verbreitungsgeschwindigkeit der rheographischen Welle. Nach dem endgültigen Beenden der Behandlung werden die erhaltenen Daten einer statistischen Bearbeitung unterzogen. Alle Kranken wurden nach Beenden des akuten Versuches in den chronischen aufgenommen.

b/ Beim chronischen Versuch wurde allen Kranken Orozetam muskulär injiziert, je Ampulle zweimal täglich um 8 Uhr und um 16.30 Uhr. Bei 9 Kranken war die zweite Orozetam-Aufnahme (um 16.30 Uhr) peroral.

Die Dauer der Behandlung betrug von 10 bis 29 Tagen. Beim chronischen Versuch wurden die rheographischen Untersuchungen vor Beginn und nach dem Beenden des Behandlungszyklus gemacht.

1. Bei 22 von 24 Kranken mit Kopfschmerzen wurde ein Nachlassen oder vollständiges Abklingen der zephalalgischen Erscheinungen beobachtet. Bei Kranken mit vasomotorischer Zephalalgie wurde das Nachlassen oder Abklingen der Kopfschmerzen bei 100 % der untersuchten Patienten noch während der Behandlung und nach Beendigung festgestellt.

2. Bei 14 von 19 Kranken wurde ein Nachlassen in unterschiedlichem Grade oder Abklingen des Schwindels festgestellt, der als eine der Hauptbeschwerden gilt.

3. 12 der untersuchten Kranken zeigten eine erhöhte geistige Arbeitsfähigkeit, verbesserte geistige Fähigkeiten, besseres Fixieren der Aufmerksamkeit.

4. Beeindruckend war die Mitteilung von 6 Kranken mit Beschwerden über einen leichten und kurzen Schlaf und über Schlaflosigkeit, daß sich schon mit der zweiten Dosis um 16.30 ihr Nachtschlaf äußerst verbessert hat, ohne jegliche zusätzliche Einnahme von Schlafmitteln oder anderen Medikamenten.

5. Zwei Kranke (Männer von 46 und 52 Jahren) teilten eine verbesserte Potenz mit.

6. Bei keinem der Kranken wurden allergische, gastrointestinale oder andere Nebenwirkungen beobachtet.

7. Die günstigen Wirkungen des Orozetams wurden hauptsächlich bei Kranken unter 60 Jahren festgestellt und dabei ohne schwere rheographische Veränderungen.

Beispiel 7

### A. Ampullen

| | |
|---|---|
| Piracetam | 20 kg |
| Orotsäure | 1 kg |
| Ethanolamin | 0,80 kg |
| Zitronensäure – bis zu einem pH-Wert von 5,5 bis 6,5 | |
| Wasser – für Injektionen bis 100 l | |

### B. Tabletten

| | | | |
|---|---|---|---|
| Piracetam | 0,05 | bis | 1,0 g |
| Orotsäure | 0,01 | bis | 0,10 g |
| mikrokristalline Zellulose | 0,05 | bis | 0,10 g |
| Laktose | 0,05 | bis | 0,10 g |
| Aerosil | 0,001 | bis | 0,008 g |
| Talk | 0,003 | bis | 0,006 g |
| Magnesiumstearat | 0,002 | bis | 0,004 g |

### C. Harte Gelatinekapsel

| | | | |
|---|---|---|---|
| Piracetam | 0,005 | bis | 0,600 g |
| Orotsäure | 0,01 | bis | 0,100 g |
| mikrokristalline Zellulose | 0,05 | bis | 0,100 g |
| Laktose | 0,05 | bis | 0,100 g |
| hochmolekulares Polymer | 0,001 | bis | 0,05 g |
| Talk | 0,004 | bis | 0,05 g |
| Magnesiumstearat | 0,002 | bis | 0,025 g |

Tabelle 1

Antihypoxischer Effekt von Piracetam und Orotsäure nach einem Modell hypobarer Hypoxie bei Mäusen

| Stoff | Dosis mg/kg | Erhöhung der Resistenz gegen Hypoxie (%) |
|---|---|---|
| Piracetam | 500 | 29 |
| Orotsäure | 25 | 5 |
| Pyrazetam + Orotsäure | 500 + 25 | 76,6 |
| Piracetam | 1000 | 37,9 |
| Orotsäure | 50 | 9,7 |
| Pyrazetam + Orotsäure | 1000 + 50 | 91,5 |

Die Ergebnisse sind in Prozenten gegenüber der entsprechenden Kontrolle angegeben.

## Patentansprüche

Antihypoxisches Mittel, insbesondere ein Mittel mit einer gehirnprotektiven und hepatoprotektiven Wirkung, dadurch gekennzeichnet, daß es 2-Oxopyrrolidin-1-azetamid und Orotsäure und/oder deren pharmazeutisch verträgliche Salze in Gewichtsverhältnissen von 500:1 bis 1:2 enthält.

## Claims

Antihypoxic medicine, in particular a medicine with a brain-protective and hepatoprotective effect, characterised in that it contains 2-oxopyrrolidine-1-acetamide and orotic acid and/or their pharmaceutically compatible salts in weight ratios of 500:1 to 1:2.

## Revendications

Agent anti-hypoxique, notamment agent ayant une activité hépatoprotectrice et cérébro-protectrice, caractérisé en ce qu'il comprend de l'oxo-2-pyrrolidinyl-1 acétamide et de l'acide orotique et/ou ses sels pharmaceutiquement acceptables dans un rapport pondéral de 500:1 à 1:2.

7